# FASCICULE DE BREVET EUROPEEN

(11) **EP 1 842 527 B1**
(45) Date de publication et mention de la délivrance du brevet: **26.06.2019**
(21) Numéro de dépôt: 07105736.8
(22) Date de dépôt: 05.04.2007
(51) Int. Cl.: A61K 8/49, A61K 8/60, A61Q 19/08, A61Q 19/00, A61K 31/70, A61Q 5/00

(54) **Utilisation de composé c-glycoside comme agent activateur et régulateur de l'immunité cutanée**
Anwendung eines Lösungsmittels mit C-Glykosid als Wirkstoff zur Anregung und Regulierung der Hautimmunität
Use of a C-glycoside compound as an agent for activating and regulating skin immunity

(30) Priorité: 07.04.2006 FR 0651265
(43) Date de publication de la demande: 10.10.2007
(73) Titulaire: L'Oréal, 75008 Paris (FR)
(72) Inventeur: Pineau, Nathalie, 95220 HERBLAY (FR); Dalko, Maria, 91190 GIF S/YVETTE (FR)
(74) Mandataire: Pillet, Anne-Helene

(56) Documents cités:
- EP-A- 0 771 795
- EP-A2- 0 260 697
- WO-A-2006/090307
- WO-A1-96/01645
- FR-A- 2 818 547
- FR-A- 2 869 317
- FR-A1- 2 818 646
- US-B1- 6 197 752

## Description

La présente invention se rapporte à l'utilisation de composés C-glycosides comme agent stimulant du système immunitaire de la peau et/ou comme immunorégulateur et pour la préparation d'une composition renfermant un milieu cosmétiquement ou pharmaceutiquement acceptable, notamment destinée à prévenir et/ou limiter l'apparition des déséquilibres immunitaires cutanés, en particulier, liés aux stress de l'environnement. Les désordres immunitaires cutanés sont des phénomènes physiologiques normaux qui apparaissent avec l'age. Ils peuvent néanmoins être accélérés par les infections de microorganismes (virus et bactéries), le stress, le vieillissement chronologique, les ultraviolets, les conditions de vie dites urbaines.

Le système immunitaire comprend un ensemble de cellules spécialisées soumises à de multiples mécanismes de contrôle assurant leur renouvellement, leur activation et leur différenciation, indispensables à un niveau normal d'immunocompétence. Le rôle du système immunitaire est de discriminer le soi du non soi pour éliminer les agents pathogènes et les tumeurs spontanées. Toute déplétion cellulaire, toute mauvaise régulation immunitaire ou tout déficit fonctionnel est susceptible de favoriser la survenue de manifestations qui vont de l'inconfort à des désordres pathologiques caractérisés par la perturbation des mécanismes de reconnaissance du soi vis-à-vis du non soi, et une plus grande sensibilité vis-à-vis des agressions microbiennes et des processus néoplasiques.

La peau est un organe de grande importance pour l'organisme et est reconnue comme l'un des principaux éléments actifs du système de défense immunitaire. Trois types de cellules épidermiques participent à ce système : les kératinocytes, les mélanocytes et les cellules de Langerhans. Ces cellules que l'on ne retrouve qu'au niveau de la peau, jouent un rôle primordial dans la réponse immunitaire et en particulier dans la présentation antigénique.

La peau saine constitue une barrière et est capable de se défendre contre les agressions extérieures, notamment chimiques, mécaniques, à ce titre un certain nombre de réactions de défense contre les facteurs environnementaux (climat, rayons ultraviolets, tabac, pollutions) et/ou les xénobiotiques (comme par exemple certains médicaments) se produisent à son niveau.

Différents facteurs, tels que les polluants atmosphériques, les détergents, les allergènes, les rayonnements UV, affectent négativement, par leur action au niveau de la peau, une variété de réponses immunes aussi bien localement au niveau du site d'exposition, qu'au niveau systémique, à des sites distants. Cette forme d'immunosuppression est notamment liée, à l'induction de cellules T suppresseur spécifiques d'antigène. L'altération de la réponse retardée est particulièrement importante car les réactions immunitaires générées par les lymphocytes T sont responsables de la protection contre de nombreuses pathologies chroniques infectieuses.

Il existe également des pathologies reposant non pas sur une insuffisance de cellules immunitaires mais sur un déséquilibre immunitaire, c'est le cas, en particulier des maladies atopiques et des maladies autoimmunes qui présentent, respectivement, un excès de lymphocytes Th-2 et un excès de lymphocytes Th-1.

La prévalence des maladies atopiques (accompagnées d'une présence excessive de lymphocytes de type Th-2 telles que la dermatite atopique, les allergies gastrointestinales, les rhinites et conjonctivites allergiques, l'asthme) et des maladies auto-immunes (accompagnées d'une présence excessive de lymphocytes de type Th-1 telles que le psoriasis, le vitiligo, la sclérodermie diffuse, le lupus érythémateux, certaines formes de pelades, la polyarthrite rhumatoïde, le diabète de type I) a augmenté progressivement durant les dernières décennies dans les sociétés occidentales.
L'explication qui est apparue la plus plausible concernant l'augmentation des affections liées à Th-2 est l'hypothèse hygiéniste qui suggère que l'augmentation rapide des eczémas atopiques est liée à la propreté actuelle des environnements et à la diminution de l'exposition aux microbes au début de la vie (Holt PG, In Nestlé Nutrition Workshop series Pediatric Program, Isolauri E et al ed, Allergic diseases and the environment, Karger AG, Basel, vol 53 pp53-68, 2004).

Au cours de la réaction de type allergique, qui peut être expliquée par une réorientation des réactions immunes du type Th-1 vers des réponses du type Th-2, l'interaction entre l'hôte sain et l'allergène est altérée. La réaction allergique s'accompagne alors d'un déséquilibre de la réponse immunitaire qui pourra alors être induite par les bactéries résidentes (Martinez FD, Respir Res : 2 :129-132, 2001).
Ces troubles atopiques sont des réactions inflammatoires chroniques et souvent systémiques d'origine complexe (facteurs génétiques et d'environnement). Dans ces pathologies, les réponses des cellules T auxiliaires de type 2 (Th-2) à des antigènes (allergènes) « inoffensifs » de l'environnement jouent un rôle déterminant dans le déclenchement des troubles allergiques (Romagnani S, Curr Opin Immunol 6 :838-846, 1994). Les cellules Th-2 expliquent l'intervention conjointe, dans le processus inflammatoire allergique des cellules B produisant des immunoglobulines E (par l'intermédiaire de la production d'interleukine IL-4 et d'IL-13), et des mastocytes (par l'intermédiaire de la production d'IL-5).

Par ailleurs, il est également important de souligner que si il y a actuellement une augmentation des pathologies allergiques liées aux cellules de type Th-2, dans le même temps, il est observé dans les pays en développement une augmentation des pathologies liées aux cellules Th-1 (maladies auto-immunes, tels que le diabète de type I, le psoriasis, le vitiligo).

Les cellules de type Th-1 ont un rôle important dans le développement de la réaction d'hypersensibilité retardée (HSR), ainsi lors de certaines maladies chroniques auto-immunes comme la polyarthrite rhumatoïdes et la thyroïdite, les lésions cutanées observées sont de type HSR, et les cellules CD4 T au sein de celles-ci sont principalement du type Th-1. Des résultats identiques ont été obtenus au cours de maladies infectieuses dues à des mycobactéries (tuberculose, lèpre), au cours de la maladie de Lyme et au cours du psoriasis.

Le vitiligo est un désordre de la dépigmentation acquis de la peau affectant 1 % de la population du monde, quelque soit la couleur de la peau. Le vitiligo est une maladie cutanée dans laquelle les mélanocytes (MCs) sont éliminés de la couche basale de l'épiderme dans les lésions. Cette disparition des mélanocytes conduit à un défaut de pigmentation. Dans les lésions de vitiligo, les mélanocytes sont détruits par des cellules T-MCs-réactives. La dépigmentation commence fréquemment pendant l'adolescence.
Par exemple, après une infection, une irradiation UV ou une agression chimique/mécanique, les mélanocytes sont endommagés. Dans des conditions de contrôle immunitaire normal, ces altérations sont contrôlées par le système immunitaire qui détruit les cellules modifiées. Dans le cas du vitiligo, ces altérations ne sont pas correctement prises en charge et elles constituent une source d'auto anticorps qui va participer à l'installation de la pathologie autoimmune.

Aussi, il apparaît qu'une thérapie qui permettrait de réorienter la réponse immunitaire « allergique » Th-2 ou « autoimmune » Th-1 vers un équilibre physiologique conduirait à des produits dont l'application topique pourrait induire une régulation des phénomènes immunitaires locaux.
La Demanderesse a maintenant mis en évidence que des composés C-glycosides de formule générale (I) étaient à la fois capables de stimuler le système immunitaire de la peau mais aussi de rétablir un déséquilibre immunitaire entre les population de lymphocytes Th-1 et Th-2 et susceptibles de provoquer des désordres auto-immuns.
Il est connu que certains sucres tels que les aldoses, les cétoses, les désoxyoses, les dérivés d'oses stimulent les défenses immunitaires (EP 0 818 201).
Il existe également des molécules O-glycosidiques ou C-glycosidiques qui modulent le système immunitaire tels que des composés C-glycolipidiques (WO 2003/105769), des fucopeptides et des dérivés amidodexoygalactose (US 5,962,660 et WO 96/29339).
Par ailleurs, des compositions cosmétiques comprenant des composés C-glycosides sont notamment connues. On peut citer en particulier le document WO2006/090307 qui divulgue des compositions de soin capillaire pour prévenir le phénomène de cheveux cassants ainsi que le document FR 2 818 646 où il est connu d'utiliser des composés C-glycosides comme agents émulsionnants et/ou tensioactifs. EP771795 et FR2818547 décrivent l'utilisation d'une partie des composés mentionnés dans ce document pour d'autres utilisations dermatologiques. EP260697 décrit différents composés pour traiter la canitie. WO9601645 décrit l'utilisation de monosaccharides peptidiques pour le traitement du Vitiligo et de maladies similaires.

La présente invention se rapporte à l'utilisation de composés de formule générale (I) : 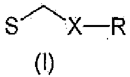 dans laquelle,
- S représente un monosaccharide ou un polysaccharide jusqu'à 20 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- la liaison S-CH₂X représente une liaison de nature C-anomèrique ;
- X représente un groupement choisi parmi: -CO-, -CHCNR₁R₂)-, -CHR'-, -C(=CHR')- ;
- R représente une chaîne alkyle, perffuoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle ladite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR'''₁R'₂, -COOR"₂, -CONHR'''₂, -CN, halogène, perfluoroalkyle, hydroffuoroalkyle et/ou au moins un radical cycloalkyle, aryle, hétérocyclique éventuellement substitués ;
- R', R₁, R₂, identiques ou différents ont la même définition que celle donnée pour R, et peuvent également représenter un hydrogène et un radical hydroxyle ;
- R'₂, R'''₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 20 atomes de carbone ;
- R'₁, R"₁, R"₂, R'''₁, identiques ou différents, représentent un atome d'hydrogène, un radical choisi parmi un radical alkyle, perfluoroalkyle et/ou hydrofluoroalkyle, linéaire ou ramifié, saturé ou insaturé, comprenant de 1 à 20 atomes de carbone ;
avec les restrictions suivantes :
- R₁ et R₂ ne pouvant pas être simultanément un radical hydroxyle ;
- R'''₁, et R'₂ ne pouvant pas être simultanément un radical hydroxyle ;
- quand S est un D-xylose et X est =CO alors R ne peut pas être un phenyle non substitué ;
- quand S est un D-glucose et X est la fonction -OH alors R ne peut pas être un p-hydroxy-phenyle ; et
- quand S est un D-glucose et X est =O alors R ne peut pas être un p-methoxy-phenyle ou un hexyle
pour prévenir et/ou traiter la canitie.

Les composés C-glycosides utilisables selon l'invention représentent une sous famille des dérivés C-glycosides décrits dans l'EP 1 345 919, ils peuvent être préparés selon le procédé décrit dans ce document.

De façon préférée, les composés utilisés selon l'invention sont tels que :
- S représente un monosaccharide ou un polysaccharide jusqu'à 5 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- R représente une chaîne alkyle, linéaire ou ramifiée, saturée ou insaturée, un cycle cycloalkyle, comprenant de 1 à 14 atomes de carbone, un radical phényle, la dite chaîne, ledit cycle ou ledit radical pouvant être éventuellement substituée par au moins un radical choisi parmi -OR'₁, -NR'''₁R'₂, -COOR"₂, -CONHR'''₂
- R', R₁, R₂, identiques ou différents ont la même définition que celle donnée pour R, et peuvent également représenter un hydrogène et un radical hydroxyle, R₁ et R₂ ne pouvant pas être simultanément un radical hydroxyle ;
- R'₂, R'''₂, identiques ou différents, représentent un atome d'hydrogène, un radical choisi parmi un radical alkyle, hydroxyle, linéaire ou ramifié, saturé ou insaturé, comprenant de 1 à 8 atomes de carbone, R'''₁, et R'₂ ne pouvant pas être simultanément un radical hydroxyle ;
- R'₁, R"₁, R"₂, R'''₁, identiques ou différents, représentent un atome d'hydrogène, un radical choisi parmi un radical alkyle, linéaire ou ramifié, saturé ou insaturé, comprenant de 1 à 8 atomes de carbone ;
la définition des autres substituant de la formule générale (I) restant inchangée.

Selon un autre variante, on préférera les composés de formule générale (I) tels que :
- S représente un monosaccharide ou un polysaccharide jusqu'à 2 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- X représente un groupement choisi parmi : -CO-, -CH(NR₁R₂)-, -CHR';
la définition des autres substituant de la formule générale (I) restant inchangée ou adoptant les valeurs préférées ci-dessus.

Avantageusement, les monosaccharides préférés sont choisi parmi le D-glucose, le D-mannose, le D-xylose, le D-lyxose, L-arabinose, le L-rhamnose, l'acide D-glucuronique, l'acide D-galacturonique, l'acide D-iduronique, la N-acétyl-D-glucosamine, la N-acétyl- D-galactosamine et désigne avantageusement le D-glucose, le D-xylose, la N-acétyl-D-glucosamine ou le L-fucose, et très préférentiellement le D-xylose.

Avantageusement encore, les polysaccharides préférés contenant jusqu'à 6 unités sucre sont choisis parmi le D-maltose, le D-cellobiose, le D-maltotriose, un disaccharide associant un acide uronique choisi parmi l'acide D-iduronique ou l'acide D-glucuronique avec une hexosamine choisi parmi la D-galactosamine, la D-glucosamine, la N-acétyl-D-galactosamine, la N-acétyl-D-glucosamine, un oligosaccharide contenant au moins un xylose avantageusement choisis parmi le xylobiose, le méthyl-β-xylobioside, le xylotriose, le xylotétraose, le xylopentaose et le xylohexaose et préférentiellement le xylobiose qui est composé de deux molécules de xylose liés par une liaison 1-4.

Parmi les dérivés C-glycosides de formule (I) utilisés selon l'invention, on préfère tout particulièrement :
Composé 1. 1-(C-β-D-xylopyranosyl)-propane-2-one ; 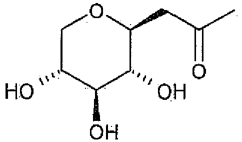
Composé 2. 1-(C-β-D-glucopyranosyl)-propane-2=one ;

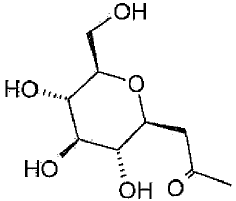

Plus particulièrement, l'utilisation des C-glycosides de formule générale (I) selon l'invention est adaptée à la préparation de la peau à l'exposition au soleil.
Ainsi, l'utilisation selon l'invention permet de prévenir et/ou limiter les effets néfastes résultant de l'exposition aux UV.

Les C-glycosides de formule générale (I) sont aussi utiles pour maintenir un équilibre entre les populations de lymphocytes Th-1 et Th-2 et/ou pour corriger un déséquilibre immunitaire lié à un excès de lymphocytes de type Th-1 ou de lymphocytes de type Th-2.
Ces composés selon l'invention pourront donc être avantageusement être utilisés pour lutter contre la canitie.

Selon un autre de ses objets, la présente invention se rapporte à l'utilisation de composés C-glycosides de formule générale (I) pour la préparation d'une composition, comprenant un milieu physiologiquement acceptable, destinée à la prévention et/ou au traitement des maladies autoimmunes cutanées.

Les maladies autoimmunes cutanées sont choisies parmi l'hypersensibilité retardée de contact, le vitiligo, la sclérodermie diffuse, le lupus érythémateux ou certaines pelades.

Par agent immunostimulant, on entend un composé dont l'administration à un organisme conduit à la prolifération des cellules immunitaires dudit organisme, par exemple, les lymphocytes.

Par agent immunorégulateur, on entend un agent capable de maintenir et/ou de rétablir un équilibre immunitaire cutané entre les populations des cellules de type Th-1 et de type Th-2, ou encore de corriger une présence excessive en cellules de type Th-1 ou de type Th-2.

Un déséquilibre immunitaire pourra notamment être mis en évidence par l'augmentation dans un organisme d'une ou plusieurs cytokines caractéristiques d'un type de lymphocyte. En effet, en plus de leur classification selon la structure de leur récepteur T, les lymphocytes de type Th-1 et de type Th-2 ont été classés selon leur profil de cytokines.
Les cytokines caractéristiques des lymphocytes de types 1 (Th-1) sont IL-2, IFN-γ, le TNF-β. Les cytokines des lymphocytes de type 2 (Th-2) sont l'IL-4, IL-5, IL-9, IL-10, IL-13

De façon plus générale, les composés C-glycosides de formule générale (I) sont utilisables comme médicament immunostimulant, chez l'homme ou chez l'animal.
Pour ce type d'utilisation, les compositions comprenant les composés C-glycosides de formule générale (I) peuvent être administrées par exemple par voie parentérale (intra-péritonéale, sous-cutanée, intra-musculaire, intra-veineuse, percutanée), par voie orale, par voie nasale, par voie conjonctivale, par voie rectale ou par voie per-linguale.
Elle peut être aussi utilisée en application locale, par exemple à l'aide de comprimés à délitement buccal, notamment dans l'immunothérapie non spécifique des maladies de la cavité buccale.

Le médicament de l'invention peut être administré à titre prophylactique, dans les différents cas ci-dessus et en particulier pour la prévention des infections récidivantes de la sphère otorhinolaryngologique (ORL), et pour la prévention des risques infectieux chez les malades chroniques.
Le médicament de l'invention est administré notamment à titre de traitement immunostimulant, dans le domaine ORL ou bronchopulmonaire (rhinopharyngites, laryngites, sinusites, angines, otites, bronchites...) ou dans le domaine dermatologique, dans le cas d'infections bactériennes, fongiques ou virales.

De préférence, le composé C-glycoside de formule générale (I) selon l'invention sera formulé dans une composition cosmétique ou pharmaceutique destinée à être appliquée topiquement sur la peau, le cuir chevelu ou les muqueuses.

Les compositions utilisées selon l'invention peuvent se présenter sous toutes les formes adaptées aux applications envisagées, notamment par voie topique, dans les domaines cosmétiques et dermatologiques.

La composition selon l'invention contient un milieu physiologiquement acceptable et un ou plusieurs composés selon l'invention en une quantité efficace pour stimuler l'immunité de la peau ou pour rééquilibrer l'équilibre entre lymphocytes Th-1 et Th-2, par exemple en une quantité allant de 0,01 à 30 % en poids et de préférence de 0,1 à 5 % en poids, par rapport au poids total de la composition.

Par milieu physiologiquement acceptable, on comprend un milieu compatible avec la peau et éventuellement avec les muqueuses, les ongles, le cuir chevelu et/ou les cheveux.

La composition selon l'invention peut avoir la forme notamment d'une solution aqueuse ou d'une dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme shampooing ou après-shampooing.

Lorsque la composition utilisable selon l'invention est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

La composition selon l'invention peut être destinée à une application cosmétique ou pharmaceutique, particulièrement dermatologique. De préférence la composition selon l'invention est destinée à une application cosmétique.

L'invention a donc également pour objet un procédé de traitement cosmétique de la peau ou du cuir chevelu, comprenant l'application topique sur la peau ou le cuir chevelu de la composition décrite précédemment.

Compte tenu des propriétés immunostimulantes et équilibrantes des composés selon l'invention, ce procédé est en particulier destiné à renforcer les défenses naturelles de la peau et améliorer l'équilibre immunitaire cutané.

Les composés C-glycosides selon l'invention seront avantageusement associés à des actifs capillaires choisis parmi :
- les anti-séborrhéiques tels que certains acides aminés soufrés, l'acide 13-cis rétinoïque, l'acétate de cyprotérone ;
- les agents de lutte contre les états squameux du cuir chevelu (pellicules) comme le zinc pyrithione, le disulfure de sélénium, le climbazole, l'acide undécylénique, le Kétoconazole, la piroctone olamine (octopirox) ou la ciclopiroctone (ciclopirox) ;

- les actifs stimulant la repousse et/ou favorisant le ralentissement de la chute des cheveux choisis parmi :
   * les esters d'acide nicotinique, dont notamment le nicotinate de tocophérol, le nicotinate de benzyle et les nicotinates d'alkyles en C₁-C₆ comme les nicotinates de méthyle ou d'hexyle ;
   * les dérivés de pyrimidine, comme le 2,4-diamino 6-piperidinopyrimidine 3-oxyde ou "Minoxidil" décrit dans les brevets US 4,139,619 et US 4,596,812 ; l'Aminexil ou 2,4 diamino pyrimidine 3 oxyde décrit dans WO96/09048 ;
   * les agents à la fois inhibiteurs de la lipoxygénase et inducteurs de la cyclo-oxygénase, ou les agents inducteurs de la cyclo-oxygénase favorisant la repousse des cheveux comme ceux décrits par la Demanderesse dans la demande de brevet européen EP 0 648 488 ;
- les agents antibiotiques tels que les macrolides, les pyranosides et les tétracyclines, et notamment l'Erythromycine ;
- la Cinnarizine, la Nimodipine et la Nifedipine ;
- des hormones, telles que l'estriol ou des analogues, ou la thyroxine et ses sels ;
- des agents antiandrogènes, tels que l'oxendolone, la spironolactone, le diéthylstilbestrol et la flutamide ;
- la cromakalim et le nicorandil.

### Exemple 1 - Mise en évidence de l'activité immunostimulante des dérivés C-glycosides de formule générale (I)

L'activité immunostimulante est testée de la façon suivante : des cellules de sang périphérique humain sont mises en culture en présence d'un milieu de culture de type RPMI supplémenté par de la L-Glutamine (2mM), de la pénicilline/streptomycine (50µg/50Ui/ml), et du sérum de veau foetal (10%). Les dérivés C-glycosides sont ajoutés à différentes concentrations (10 à 0.05mM) ainsi que la phytohemagglutine (PHA à 5 *G/ml), contrôle positif de la prolifération lymphocytaire. Après 3 jours de culture la prolifération est révélée par un marquage au BrdU.

Les résultats obtenus sont les suivants :

| Actif | % de stimulation par rapport au contrôle | | | | | |
|---|---|---|---|---|---|---|
| Concentrations (mM) → | 10 | 5 | 1 | 0.5 | 0.1 | 0.05 |
| **Composé 1** : 1-(C-β-D-xylopyranosyl)-propane-2-one | 74* | 106* | 131 | 84 | 115 | 108 |
| **Composé 2** : 1-(C-β-D-glucopyranosyl)-propane-2-one | 224 | 227 | 123 | 90 | 76 | 64 |

| | | | | | | |
|---|---|---|---|---|---|---|
| * cytotoxicité | | | | | | |

Tous les dérivés testés présentent une forte capacité de prolifération des lymphocytes humains.
Le composés 1 et 2 stimulent la prolifération des lymphocytes humains à partir de 1 mM, ces composés présentent donc une activité immunostimulante.

### EXEMPLE 2 - Formulations

**Gel pour le soin du visage**

| | |
|---|---|
| Composé 2 | 0,05 % |
| Polymère épaississant | 1,00 % |
| Antioxydant | 0,05 % |
| Isopropanol | 40,00 % |
| Conservateur | 0,30 % |
| Eau | qsp 100 % |

**Lotion pour le visage des peaux hyper réactives**

| | |
|---|---|
| Composé 1 | 0,50 |
| Gluconate de magnésium | 3,00 |
| Antioxydant | 0,05 |
| Isopropanol | 40,0 |
| Conservateur | 0,30 |
| Eau | qsp 100 % |

## Revendications

1. Utilisation cosmétique d'au moins un composé de formule générale (I) : 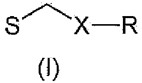 dans laquelle,
- S représente un monosaccharide ou un polysaccharide jusqu'à 20 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- la liaison S-CH₂X représente une liaison de nature C-anomèrique ;
- X représente un groupement choisi parmi: -CO-, -CH(NR₁R₂)-, -CHR'-, -C(=CHR')-;
- R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR'''₁R'₂, -COOR"₂, -CONHR'''₂, -CN, halogène, perfluoroalkyle, hydrofluoroalkyle et/ou au moins un radical cycloalkyle, aryle, hétérocyctique éventuellement substitués ;
- R', R₁, R₂, identiques ou différents ont la même définition que celle donnée pour R, et peuvent également représenter un hydrogène et un radical hydroxyle ;
- R'₂, R'''₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 20 atomes de carbone ;
- R'₁, R"₁, R"₂, R'''₁, identiques ou différents, représentent un atome d'hydrogène, un radical choisi parmi un radical alkyle, perfluoroalkyle et/ou hydrofluoroalkyle, linéaire ou ramifié, saturé ou insaturé, comprenant de 1 à 20 atomes de carbone ;
à l'exclusion des composés tels que :
- R₁ et R₂ ne pouvant pas être simultanément un radical hydroxyle ;
- R'''₁ et R'₂ ne pouvant pas être simultanément un radical hydroxyle ;
- quand S est un D-xylose et X est =CO alors R ne peut pas être un phenyle non substitué ;
- quand S est un D-glucose et X est la fonction -OH alors R ne peut pas être un p-hydroxy-phenyle ; et
- quand S est un D-glucose et X est =O alors R ne peut pas être un p-methoxy-phenyle ou un hexyle
pour prévenir et/ou traiter la canitie.

2. Utilisation selon la revendication 1, **caractérisée en ce que** ledit Composé de formule générale (I) est associé à au moins un actif capillaire choisi parmi les anti-séborrhéiques ; les agents de lutte contre les états squameux du cuir chevelu ; les actifs stimulant la repousse et/ou favorisant le ralentissement de la chute des cheveux ; les agents antibiotiques ; la Cinnarizine, la Nimodipine et la Nifedipine ; l'estriol ou des analogues, la thyroxine et ses sels ; l'oxendolone, la spironolactone, le diéthylstilbestrol la flutamide ; la cromakalim et le nicorandil..

3. Utilisation d'au moins un composé de formule générale (I) : 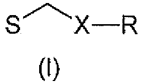 dans laquelle,
- S représente un monosaccharide ou un polysaccharide jusqu'à 20 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- la liaison S-CH₂X représente une liaison de nature C-anomèrique ;
- X représente un groupement choisi parmi: -CO-, -CHCNR₁R₂)-, -CHR'-, -C(=CHR')- ;
- R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR'''₁R'₂, -COOR"₂, -CONHR'''₂, -CN, halogène, perfluoroalkyle, hydrofluoroalkyle et/ou au moins un radical cycloalkyle, aryle, hétérocyclique éventuellement substitués ;
- R', R₁, R₂, identiques ou différents ont la même définition que celle donnée pour R, et peuvent également représenter un hydrogène et un radical hydroxyle ;
- R'₂, R'''₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 20 atomes de carbone ;
- R'₁, R"₁, R"₂, R'''₁, identiques ou différents, représentent un atome d'hydrogène, un radical choisi parmi un radical alkyle, perfluoroalkyle et/ou hydrofluoroalkyle, linéaire ou ramifié, saturé ou insaturé, comprenant de 1 à 20 atomes de carbone ;
à l'exclusion des composés tels que :
- R₁ et R₂ ne pouvant pas être simultanément un radical hydroxyle ;
- R'''₁ et R'₂ ne pouvant pas être simultanément un radical hydroxyle ;
- quand S est un D-xylose et X est =CO alors R ne peut pas être un phenyle non substitué ;
- quand S est un D-glucose et X est la fonction -OH alors R ne peut pas être un p-hydroxy-phenyle ; et
- quand S est un D-glucose et X est =O alors R ne peut pas être un p-methoxy-phenyle ou un hexyle
pour la préparation d'une composition comprenant un milieu physiologiquement acceptable destinée à la prévention et/ou au traitement des maladies autoimmunes cutanées **caractérisée en ce que** les maladies autoimmunes cutanées sont choisies parmi l'hypersensibilité retardée de contact, le vitiligo, la sclérodermie diffuse, le lupus érythémateux ou certaines pelades.

4. Utilisation selon la revendication précédente, **caractérisée en ce que** ladite composition est destinée à être appliquée topiquement sur la peau, les muqueuses ou le cuir chevelu.

5. Utilisation selon l'une quelconque des revendications précédentes, **caractérisée en ce que** les composés de formule générale (I) sont tels que S représente un monosaccharide ou un polysaccharide jusqu'à 5 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée.

6. Utilisation selon la revendication 5, **caractérisée en ce que** les polysaccharides préférés contenant jusqu'à 6 unités sucre sont choisis parmi le D-maltose, le D-cellobiose, le D-maltotriose, un disaccharide associant un acide uronique choisi parmi l'acide D-iduronique ou l'acide D-glucuronique avec une hexosamine choisi parmi la D-galactosamine, la D-glucosamine, la N-acétyl-D-galactosamine, la N-acétyl-D-glucosamine, un oligosaccharide contenant au moins un xylose avantageusement choisis parmi le xylobiose, le méthyl-β-xylobioside, le xylotriose, le xylotétraose, le xylopentaose et le xylohexaose et préférentiellement le xylobiose qui est composé de deux molécules de xylose liés par une liaison 1-4.

7. Utilisation selon la revendication 5, **caractérisée en ce que** S représente un monosaccharide ou un polysaccharide jusqu'à 2 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée.

8. Utilisation selon la revendication 7, **caractérisée en ce que** les monosaccharides préférés sont choisi parmi le D-glucose, le D-mannose, le D-xylose, le D-lyxose, L-arabinose, le L-rhamnose, l'acide D-glucuronique, l'acide D-galacturonique, l'acide D-iduronique, la N-acétyl-D-glucosamine, la N-acétyl- D-galactosamine et désigne avantageusement le D-glucose, le D-xylose, la N-acétyl-D-glucosamine ou le L-fucose, et très préférentiellement le D-xylose.

9. Utilisation selon l'une quelconque des revendications précédentes, **caractérisée en ce que** R représente une chaîne alkyle, linéaire ou ramifiée, saturée ou insaturée, un cycle cycloalkyle, comprenant de 1 à 14 atomes de carbone, un radical phényle, ladite chaîne, ledit cycle ou ledit radical pouvant être éventuellement substituée par au moins un radical choisi parmi -OR'₁, -NR'''₁R'₂, -COOR"₂, -CONHR'''₂.

10. Utilisation selon l'une quelconque des revendications précédentes, **caractérisée en ce que** le composé de formule générale (I) est choisi parmi :
Composé 1. 1-(C-β-D-xylopyranosyl)-propane-2-one ;
Composé 2. 1-(C-β-D-glucopyranosyl)-propane-2-one.

11. Composition comprenant au moins un composé de formule générale (I) : 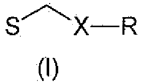 dans laquelle,
- S représente un monosaccharide ou un polysaccharide jusqu'à 20 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- la liaison S-CH₂X représente une liaison de nature C-anomèrique ;
- X représente un groupement choisi parmi: -CO-, -CH(NR₁R₂)-, -CHR'-, -C(=CHR')- ;
- R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR'''₁R'₂, -COOR"₂, -CONHR'''₂, -CN, halogène, perfluoroalkyle, hydrofluoroalkyle et/ou au moins un radical cycloalkyle, aryle, hétérocyclique éventuellement substitués ;
- R', R₁, R₂, identiques ou différents ont la même définition que celle donnée pour R, et peuvent également représenter un hydrogène et un radical hydroxyle ;
- R'₂, R'''₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 20 atomes de carbone ;
- R'₁, R"₁, R"₂, R'''₁, identiques ou différents, représentent un atome d'hydrogène, un radical choisi parmi un radical alkyle, perfluoroalkyle et/ou hydrofluoroalkyle, linéaire ou ramifié, saturé ou insaturé, comprenant de 1 à 20 atomes de carbone ;
à l'exclusion des composés tels que :
- R₁ et R₂ ne pouvant pas être simultanément un radical hydroxyle ;
- R'''₁ et R'₂ ne pouvant pas être simultanément un radical hydroxyle ;
- quand S est un D-xylose et X est =CO alors R ne peut pas être un phenyle non substitué ;
- quand S est un D-glucose et X est la fonction -OH alors R ne peut pas être un p-hydroxyphenyle ; et
- quand S est un D-glucose et X est =O alors R ne peut pas être un p-methoxy-phenyle ou un hexyle
et au moins un actif capillaire choisi parmi les anti-séborrhéiques ; les agents de lutte contre les états squameux du cuir chevelu ; les actifs stimulant la repousse et/ou favorisant le ralentissement de la chute des cheveux choisis parmi les esters d'acide nicotinique, les dérivés de pyrimidine, les agents à la fois inhibiteurs de la lipoxygénase et inducteurs de la cyclo-oxygénase, et les agents inducteurs de la cyclo-oxygénase favorisant la repousse des cheveux ; les agents antibiotiques ; la cinnarazine ; la nimodipine ; la nifédipine ; l'estriol ou des analogues ; la thyroxine et ses sels ; l'oxendolone, la spironolactone, le diéthylstilbestrol et la flutamide ; la cromakalim et le nicorandil.

## Patentansprüche

1. Kosmetische Verwendung mindestens einer Verbindung der allgemeinen Formel (I): 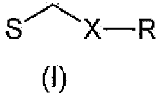 wobei
- S für ein Monosaccharid oder ein Polysaccharid mit bis zu 20 Zuckereinheiten in Pyranose- und/oder Furanose-Form und der L- und/oder D-Reihe steht, wobei das Mono- oder Polysaccharid mindestens eine Hydroxylfunktion, die frei sein muss, und/oder gegebenenfalls eine oder mehrere gegebenenfalls geschützte Aminfunktionen aufweist;
- die Bindung S-CH₂X für eine C-anomere Bindung steht;
- X für eine Gruppe, die aus -CO-, -CH(NR₁R₂)-, -CHR'-, -C(=CHR^{r})- ausgewählt ist, steht;
- R für eine gesättigte oder ungesättigte, lineare oder verzweigte Alkyl-, Perfluoralkyl- oder Hydrofluoralkylkette, einen Cycloalkyl-, Cycloperfluoralkyl- oder Cyclohydrofluoralkylring mit 1 bis 18 Kohlenstoffatomen oder einen Phenylrest steht, wobei die Kette, der Ring bzw. der Rest gegebenenfalls durch ein oder mehrere Heteroatome, die aus Sauerstoff, Schwefel, Stickstoff und Silicium ausgewählt sind, unterbrochen und gegebenenfalls durch mindestens einen Rest, der aus -OR'₁, -SR"₁, -NR'''₁R'₂, -COOR"₂, -CONHR'''₂, -CN, Halogen, Perfluoralkyl und Hydrofluoralkyl ausgewählt ist, und/oder mindestens einen gegebenenfalls substituierten Cycloalkyl-, Aryl- oder Heterocyclylrest substituiert sein kann;
- R', R₁ und R₂, die gleich oder verschieden sind, der für R angegebenen Definition entsprechen und außerdem für einen Wasserstoff und einen Hydroxylrest stehen können;
- R'₂ und R'''₂, die gleich oder verschieden sind, für ein Wasserstoffatom oder einen Rest, der aus einem gesättigten oder ungesättigten, linearen oder verzweigten Alkyl-, Hydroxyl-, Perfluoralkyl- und/oder Hydrofluoralkylrest mit 1 bis 20 Kohlenstoffatomen ausgewählt ist, stehen;
- R'₁, R"₁, R"₂ und R'''₁, die gleich oder verschieden sind, für ein Wasserstoffatom oder einen Rest, der aus einem gesättigten oder ungesättigten, linearen oder verzweigten Alkyl-, Perfluoralkyl- und/oder Hydrofluoralkylrest mit 1 bis 20 Kohlenstoffatomen ausgewählt ist, stehen;
mit Ausnahme von Verbindungen derart, dass:
- R₁ und R₂ nicht gleichzeitig für einen Hydroxylrest stehen können;
- R'''₁ und R'₂ nicht gleichzeitig für einen Hydroxylrest stehen können;
- dann, wenn S für eine D-Xylose steht und X für =CO steht, R nicht für ein unsubstituiertes Phenyl stehen kann;
- dann, wenn S für eine D-Glucose steht und X für die Funktion -OH steht, R nicht für ein p-Hydroxyphenyl stehen kann; und
- dann, wenn S für eine D-Glucose steht und X für =O steht, R nicht für ein p-Methoxyphenyl oder ein Hexyl stehen kann;
zur Prävention und/oder Behandlung von Canities.

2. Verwendung nach Anspruch 1, **dadurch gekennzeichnet, dass** die Verbindung der allgemeinen Formel (I) mit mindestens einem Haarwirkstoff, der aus Antisebborhoika; Mitteln zur Bekämpfung von üppigen Zuständen der Kopfhaut; Wirkstoffen zur Stimulierung des Nachwachsens und/oder Förderung der Verlangsamung des Ausfallens der Haare; Antibiotika; Cinnarizin, Nimodipin und Nifedipin; Estriol oder Analogen, Thyroxin und Salzen davon; Oxendolon, Spironolacton, Diethylstilbestrol, Flutamid; Cromakalim und Nicorandil ausgewählt ist; kombiniert ist.

3. Verwendung mindestens einer Verbindung der allgemeinen Formel (I): 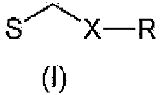 wobei
- S für ein Monosaccharid oder ein Polysaccharid mit bis zu 20 Zuckereinheiten in Pyranose- und/oder Furanose-Form und der L- und/oder D-Reihe steht, wobei das Mono- oder Polysaccharid mindestens eine Hydroxylfunktion, die frei sein muss, und/oder gegebenenfalls eine oder mehrere gegebenenfalls geschützte Aminfunktionen aufweist;
- die Bindung S-CH₂X für eine C-anomere Bindung steht;
- X für eine Gruppe, die aus -CO-, -CH(NR₁R₂)-, -CHR'-, -C(=CHR^{r})- ausgewählt ist, steht;
- R für eine gesättigte oder ungesättigte, lineare oder verzweigte Alkyl-, Perfluoralkyl- oder Hydrofluoralkylkette, einen Cycloalkyl-, Cycloperfluoralkyl- oder Cyclohydrofluoralkylring mit 1 bis 18 Kohlenstoffatomen oder einen Phenylrest steht, wobei die Kette, der Ring bzw. der Rest gegebenenfalls durch ein oder mehrere Heteroatome, die aus Sauerstoff, Schwefel, Stickstoff und Silicium ausgewählt sind, unterbrochen und gegebenenfalls durch mindestens einen Rest, der aus -OR'₁, -SR"₁, -NR'''₁R'₂, -COOR"₂, -CONHR'''₂, -CN, Halogen, Perfluoralkyl und Hydrofluoralkyl ausgewählt ist, und/oder mindestens einen gegebenenfalls substituierten Cycloalkyl-, Aryl- oder Heterocyclylrest substituiert sein kann;
- R', R₁ und R₂, die gleich oder verschieden sind, der für R angegebenen Definition entsprechen und außerdem für einen Wasserstoff und einen Hydroxylrest stehen können;
- R'₂ und R'''₂, die gleich oder verschieden sind, für ein Wasserstoffatom oder einen Rest, der aus einem gesättigten oder ungesättigten, linearen oder verzweigten Alkyl-, Hydroxyl-, Perfluoralkyl- und/oder Hydrofluoralkylrest mit 1 bis 20 Kohlenstoffatomen ausgewählt ist, stehen;
- R'₁, R"₁, R"₂ und R'''₁, die gleich oder verschieden sind, für ein Wasserstoffatom oder einen Rest, der aus einem gesättigten oder ungesättigten, linearen oder verzweigten Alkyl-, Perfluoralkyl- und/oder Hydrofluoralkylrest mit 1 bis 20 Kohlenstoffatomen ausgewählt ist, stehen;
mit Ausnahme von Verbindungen derart, dass:
- R₁ und R₂ nicht gleichzeitig für einen Hydroxylrest stehen können;
- R'''₁ und R'₂ nicht gleichzeitig für einen Hydroxylrest stehen können;
- dann, wenn S für eine D-Xylose steht und X für =CO steht, R nicht für ein unsubstituiertes Phenyl stehen kann;
- dann, wenn S für eine D-Glucose steht und X für die Funktion -OH steht, R nicht für ein p-Hydroxyphenyl stehen kann; und
- dann, wenn S für eine D-Glucose steht und X für =O steht, R nicht für ein p-Methoxyphenyl oder ein Hexyl stehen kann;
zur Herstellung einer Zusammensetzung, die ein physiologisch unbedenkliches Medium umfasst, für die Prävention und/oder Behandlung von kutanen Autoimmunerkrankungen, **dadurch gekennzeichnet, dass** die kutanen Autoimmunerkrankungen aus verzögerter Kontaktüberempfindlichkeit, Vitiligo, diffuser Sklerodermie, Lupus erythematodes oder bestimmten Peladen ausgewählt sind.

4. Verwendung nach dem vorhergehenden Anspruch, **dadurch gekennzeichnet, dass** die Zusammensetzung für die topische Anwendung auf der Haut, den Schleimhäuten oder der Kopfhaut vorgesehen ist.

5. Verwendung nach einem der vorhergehenden Ansprüche, **dadurch gekennzeichnet, dass** die Verbindungen der allgemeinen Formel (I) derart beschaffen sind, dass S für ein Monosaccharid oder ein Polysaccharid mit bis zu 5 Zuckereinheiten in Pyranose- und/oder Furanose-Form und der L- und/oder D-Reihe steht, wobei das Mono- oder Polysaccharid mindestens eine Hydroxylfunktion, die frei sein muss, und/oder gegebenenfalls eine oder mehrere gegebenenfalls geschützte Aminfunktionen aufweist.

6. Verwendung nach Anspruch 5, **dadurch gekennzeichnet, dass** die bevorzugten Polysaccharide bis zu 6 Zuckereinheiten enthalten, die aus D-Maltose, D-Cellobiose, D-Maltotriose, einem Disaccharid, in dem eine Uronsäure, die aus D-Iduronsäure oder D-Glucuronsäure ausgewählt ist, mit einem Hexosamin, das aus D-Galactosamin, D-Glucosamin, N-Acetyl-D-galactosamin und N-Acetyl-D-glucosamin ausgewählt ist, kombiniert ist, und einem Oligosaccharid, das mindestens eine Xylose enthält, vorteilhafterweise ausgewählt aus Xylobiose, Methyl-β-xylobiosid, Xylotriose, Xylotetraose, Xylopentaose und Xylohexaose und vorzugsweise Xylobiose, die aus zwei über eine 1,4-Bindung verknüpften Xylose-Molekülen besteht, ausgewählt sind.

7. Verwendung nach Anspruch 5, **dadurch gekennzeichnet, dass** S für ein Monosaccharid oder ein Polysaccharid mit bis zu 2 Zuckereinheiten in Pyranose- und/oder Furanose-Form und der L- und/oder D-Reihe steht, wobei das Mono- oder Polysaccharid mindestens eine Hydroxylfunktion, die frei sein muss, und/oder gegebenenfalls eine oder mehrere gegebenenfalls geschützte Aminfunktionen aufweist.

8. Verwendung nach Anspruch 7, **dadurch gekennzeichnet, dass** die bevorzugten Monosaccharide aus D-Glucose, D-Mannose, D-Xylose, D-Lyxose, L-Arabinose, L-Rhamnose, D-Glucuronsäure, D-Galacturonsäure, D-Iduronsäure, N-Acetyl-D-glucosamin und N-Acetyl-D-galactosamin ausgewählt sind und vorzugsweise für D-Glucose, D-Xylose, N-Acetyl-D-glucosamin oder L-Fucose und ganz besonders bevorzugt D-Xylose stehen.

9. Verwendung nach einem der vorhergehenden Ansprüche, **dadurch gekennzeichnet, dass** R für eine gesättigte oder ungesättigte, lineare oder verzweigte Alkylkette, einen Cycloalkylring mit 1 bis 14 Kohlenstoffatomen oder einen Phenylrest steht, wobei die Kette, der Ring bzw. der Rest gegebenenfalls durch mindestens einen Rest, der aus -OR'₁, -NR'''₁R'₂, -COOR"₂ und -CONHR'''₂ ausgewählt ist, substituiert sein kann.

10. Verwendung nach einem der vorhergehenden Ansprüche, **dadurch gekennzeichnet, dass** die Verbindung der allgemeinen Formel (I) ausgewählt ist aus:
Verbindung 1. 1-(C-β-D-Xylopyranosyl)propan-2-on;
Verbindung 2. 1-(C-β-D-Glucopyranosyl)propan-2-on.

11. Zusammensetzung, umfassend mindestens eine Verbindung der allgemeinen Formel (I): 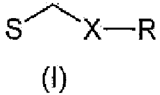 wobei
- S für ein Monosaccharid oder ein Polysaccharid mit bis zu 20 Zuckereinheiten in Pyranose- und/oder Furanose-Form und der L- und/oder D-Reihe steht, wobei das Mono- oder Polysaccharid mindestens eine Hydroxylfunktion, die frei sein muss, und/oder gegebenenfalls eine oder mehrere gegebenenfalls geschützte Aminfunktionen aufweist;
- die Bindung S-CH₂X für eine C-anomere Bindung steht;
- X für eine Gruppe, die aus -CO-, -CH(NR₁R₂)-, -CHR'-, -C(=CHR^{r})- ausgewählt ist, steht;
- R für eine gesättigte oder ungesättigte, lineare oder verzweigte Alkyl-, Perfluoralkyl- oder Hydrofluoralkylkette, einen Cycloalkyl-, Cycloperfluoralkyl- oder Cyclohydrofluoralkylring mit 1 bis 18 Kohlenstoffatomen oder einen Phenylrest steht, wobei die Kette, der Ring bzw. der Rest gegebenenfalls durch ein oder mehrere Heteroatome, die aus Sauerstoff, Schwefel, Stickstoff und Silicium ausgewählt sind, unterbrochen und gegebenenfalls durch mindestens einen Rest, der aus -OR'₁, -SR"₁, -NR'''₁R'₂, -COOR"₂, -CONHR'''₂, -CN, Halogen, Perfluoralkyl und Hydrofluoralkyl ausgewählt ist, und/oder mindestens einen gegebenenfalls substituierten Cycloalkyl-, Aryl- oder Heterocyclylrest substituiert sein kann;
- R', R₁ und R₂, die gleich oder verschieden sind, der für R angegebenen Definition entsprechen und außerdem für einen Wasserstoff und einen Hydroxylrest stehen können;
- R'₂ und R'''₂, die gleich oder verschieden sind, für ein Wasserstoffatom oder einen Rest, der aus einem gesättigten oder ungesättigten, linearen oder verzweigten Alkyl-, Hydroxyl-, Perfluoralkyl- und/oder Hydrofluoralkylrest mit 1 bis 20 Kohlenstoffatomen ausgewählt ist, stehen;
- R'₁, R"₁, R"₂ und R'''₁, die gleich oder verschieden sind, für ein Wasserstoffatom oder einen Rest, der aus einem gesättigten oder ungesättigten, linearen oder verzweigten Alkyl-, Perfluoralkyl- und/oder Hydrofluoralkylrest mit 1 bis 20 Kohlenstoffatomen ausgewählt ist, stehen;
mit Ausnahme von Verbindungen derart, dass:
- R₁ und R₂ nicht gleichzeitig für einen Hydroxylrest stehen können;
- R'''₁ und R'₂ nicht gleichzeitig für einen Hydroxylrest stehen können;
- dann, wenn S für eine D-Xylose steht und X für =CO steht, R nicht für ein unsubstituiertes Phenyl stehen kann;
- dann, wenn S für eine D-Glucose steht und X für die Funktion -OH steht, R nicht für ein p-Hydroxyphenyl stehen kann; und
- dann, wenn S für eine D-Glucose steht und X für =O steht, R nicht für ein p-Methoxyphenyl oder ein Hexyl stehen kann;
und mindestens einen Haarwirkstoff, der aus Antisebborhoika; Mitteln zur Bekämpfung von üppigen Zuständen der Kopfhaut; Wirkstoffen zur Stimulierung des Nachwachsens und/oder Förderung der Verlangsamung des Ausfallens der Haare, die aus Nicotinsäureestern, Pyrimidinderivaten, Mittel, die gleichzeitig Lipoxygenaseinhibitoren und Cyclooxygenaseinduktetoren sind, und cyclooxygenaseinduzierenden Mitteln, die das Nachwechsen der Haare fördern, ausgewählt sind; Antibiotika; Cinnarizin; Nimodipin; Nifedipin; Estriol oder Analogen; Thyroxin und Salzen davon; Oxendolon, Spironolacton, Diethylstilbestrol, Flutamid; Cromakalim und Nicorandil ausgewählt ist.

## Claims

1. Cosmetic use of at least one compound of general formula (I) : 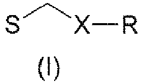 in which,
- S represents a monosaccharide or a polysaccharide of up to 20 sugar units, in pyranose and/or furanose form, and of the L and/or D series, said mono- or polysaccharide having at least one hydroxyl function that must be free and/or optionally one or more optionally protected amine functions;
- the S-CH₂X bond represents a bond of C-anomeric nature;
- X represents a group chosen from: -CO-, -CH(NR₁R₂)-, -CHR'- and -C(=CHR')-;
- R represents a linear or branched, saturated or unsaturated alkyl, perfluoroalkyl or hydrofluoroalkyl chain, or a cycloalkyl, cycloperfluoroalkyl or cyclohydrofluoroalkyl ring, containing from 1 to 18 carbon atoms, or a phenyl radical, it being possible for said chain, said ring or said radical to be optionally interrupted with one or more heteroatoms chosen from oxygen, sulfur, nitrogen and silicon, and optionally substituted with at least one radical chosen from -OR'₁-, -SR"₁, -NR'''₁R'₂, -COOR"₂, -CONHR'''₂, -CN, halogen, perfluoroalkyl and hydrofluoroalkyl, and/or at least one cycloalkyl, aryl or heterocyclic radical, optionally substituted;
- R', R₁ and R₂, which may be identical or different, have the same definition as that given for R, and can also represent a hydrogen and a hydroxyl radical;
- R'₂ and R'''₂, which may be identical or different, represent a hydrogen atom, or a radical chosen from a hydroxyl radical and a linear or branched, saturated or unsaturated alkyl, perfluoroalkyl and/or hydrofluoroalkyl radical containing from 1 to 20 carbon atoms;
- R'₁, R"₁, R"₂ and R'''₁, which may be identical or different, represent a hydrogen atom, or a radical chosen from a linear or branched, saturated or unsaturated alkyl, perfluoroalkyl and/or hydrofluoroalkyl radical containing from 1 to 20 carbon atoms;
with the exclusion of compounds such that:
- R₁ and R₂ cannot simultaneously be a hydroxyl radical;
- R'''₁ and R'₂ cannot simultaneously be a hydroxyl radical;
- when S is a D-xylose and X is =CO, then R cannot be an unsubstituted phenyl;
- when S is a D-glucose and X is the function -OH, then R cannot be a p-hydroxyphenyl; and
- when S is a D-glucose and X is =O, then R cannot be a p-methoxyphenyl or a hexyl;
for preventing and/or treating hair turning white or grey prematurely.

2. Use according to Claim 1, **characterized in that** said compound of general formula (I) is combined with at least one active agent for the hair chosen from anti-seborrhoeic agents; agents for combating squamous states of the scalp; active agents for stimulating hair regrowth and/or promoting the slowing down of hair loss; antibiotics; cinnarizine; nimodipine and nifedipine; estriol or the like, thyroxine and salts thereof; oxendolone, spironolactone, diethylstilbestrol, flutamide; cromakalim and nicorandil.

3. Use of at least one compound of general formula (I): 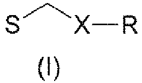 in which,
- S represents a monosaccharide or a polysaccharide of up to 20 sugar units, in pyranose and/or furanose form, and of the L and/or D series, said mono- or polysaccharide having at least one hydroxyl function that must be free and/or optionally one or more optionally protected amine functions;
- the S-CH₂X bond represents a bond of C-anomeric nature;
- X represents a group chosen from: -CO-, -CH(NR₁R₂)-, -CHR'- and -C(=CHR')-;
- R represents a linear or branched, saturated or unsaturated alkyl, perfluoroalkyl or hydrofluoroalkyl chain, or a cycloalkyl, cycloperfluoroalkyl or cyclohydrofluoroalkyl ring, containing from 1 to 18 carbon atoms, or a phenyl radical, it being possible for said chain, said ring or said radical to be optionally interrupted with one or more heteroatoms chosen from oxygen, sulfur, nitrogen and silicon, and optionally substituted with at least one radical chosen from -OR'₁-, -SR"₁, -NR'''₁R'₂, -COOR"₂, -CONHR'''₂, -CN, halogen, perfluoroalkyl and hydrofluoroalkyl, and/or at least one cycloalkyl, aryl or heterocyclic radical, optionally substituted;
- R', R₁ and R₂, which may be identical or different, have the same definition as that given for R, and can also represent a hydrogen and a hydroxyl radical;
- R'₂ and R'''₂, which may be identical or different, represent a hydrogen atom, or a radical chosen from a hydroxyl radical and a linear or branched, saturated or unsaturated alkyl, perfluoroalkyl and/or hydrofluoroalkyl radical containing from 1 to 20 carbon atoms;
- R'₁, R"₁, R"₂ and R'''₁, which may be identical or different, represent a hydrogen atom, or a radical chosen from a linear or branched, saturated or unsaturated alkyl, perfluoroalkyl and/or hydrofluoroalkyl radical containing from 1 to 20 carbon atoms;
with the exclusion of compounds such that:
- R₁ and R₂ cannot simultaneously be a hydroxyl radical;
- R'''₁ and R'₂ cannot simultaneously be a hydroxyl radical;
- when S is a D-xylose and X is =CO, then R cannot be an unsubstituted phenyl;
- when S is a D-glucose and X is the function -OH, then R cannot be a p-hydroxyphenyl; and
- when S is a D-glucose and X is =O, then R cannot be a p-methoxyphenyl or a hexyl;
for preparing a composition, comprising a physiologically acceptable medium, for use in the prevention and/or treatment of cutaneous autoimmune diseases,
**characterized in that** the cutaneous autoimmune diseases are chosen from delayed contact hypersensitivity, vitiligo, diffuse scleroderma, lupus erythematous or certain forms of alopecia.

4. Use according to the preceding claim, **characterized in that** said composition is intended to be applied topically to the skin, the mucous membranes or the scalp.

5. Use according to any one of the preceding claims, **characterized in that** the compounds of general formula (I) are such that S represents a monosaccharide or a polysaccharide of up to 5 sugar units, in pyranose and/or furanose form, and of the L and/or D series, said mono- or polysaccharide having at least one hydroxyl function that must be free and/or optionally one or more optionally protected amine functions.

6. Use according to Claim 5, **characterized in that** the preferred polysaccharides containing up to 6 sugar units are chosen from D-maltose, D-cellobiose, D-maltotriose, a disaccharide combining a uronic acid chosen from D-iduronic acid or D-glucuronic acid, with a hexosamine chosen from D-galactosamine, D-glucosamine, N-acetyl-D-galactosamine and N-acetyl-D-glucosamine, and an oligosaccharide containing at least one xylose advantageously chosen from xylobiose, methyl-β-xylobioside, xylotriose, xylotetraose, xylopentaose and xylohexaose, and preferably xylobiose, which is composed of two xylose molecules linked by a 1,4-linkage.

7. Use according to Claim 5, **characterized in that** S represents a monosaccharide or a polysaccharide of up to 2 sugar units, in pyranose and/or furanose form, and of the L and/or D series, said mono- or polysaccharide having at least one hydroxyl function that must be free and/or optionally one or more optionally protected amine functions.

8. Use according to Claim 7, **characterized in that** the preferred monosaccharides are chosen from D-glucose, D-mannose, D-xylose, D-lyxose, L-arabinose, L-rhamnose, D-glucuronic acid, D-galacturonic acid, D-iduronic acid, N-acetyl-D-glucosamine and N-acetyl-D-galactosamine, and advantageously denote D-glucose, D-xylose, N-acetyl-D-glucosamine or L-fucose, and very preferably D-xylose.

9. Use according to any one of the preceding claims, **characterized in that** R represents a linear or branched, saturated or unsaturated alkyl chain, or a cycloalkyl ring, containing from 1 to 14 carbon atoms, or a phenyl radical, it being possible for said chain, said ring or said radical to be optionally substituted with at least one radical chosen from -OR'₁, -NR'''₁R'₂, -COOR"₂ and -CONHR'''₂.

10. Use according to any one of the preceding claims, **characterized in that** the compound of general formula (I) is chosen from:
Compound 1. 1-(C-β-D-Xylopyranosyl)propan-2-one;
Compound 2. 1-(C-β-D-Glucopyranosyl)propan-2-one.

11. Composition comprising at least one compound of general formula (I): 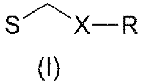 in which,
- S represents a monosaccharide or a polysaccharide of up to 20 sugar units, in pyranose and/or furanose form, and of the L and/or D series, said mono- or polysaccharide having at least one hydroxyl function that must be free and/or optionally one or more optionally protected amine functions;
- the S-CH₂X bond represents a bond of C-anomeric nature;
- X represents a group chosen from: -CO-, -CH(NR₁R₂)-, -CHR'- and -C(=CHR')-;
- R represents a linear or branched, saturated or unsaturated alkyl, perfluoroalkyl or hydrofluoroalkyl chain, or a cycloalkyl, cycloperfluoroalkyl or cyclohydrofluoroalkyl ring, containing from 1 to 18 carbon atoms, or a phenyl radical, it being possible for said chain, said ring or said radical to be optionally interrupted with one or more heteroatoms chosen from oxygen, sulfur, nitrogen and silicon, and optionally substituted with at least one radical chosen from -OR'₁, -SR"₁, -NR'''₁R'₂, -COOR"₂, -CONHR'''₂, -CN, halogen, perfluoroalkyl and hydrofluoroalkyl, and/or at least one cycloalkyl, aryl or heterocyclic radical, optionally substituted;
- R', R₁ and R₂, which may be identical or different, have the same definition as that given for R, and can also represent a hydrogen and a hydroxyl radical;
- R'₂ and R'''₂, which may be identical or different, represent a hydrogen atom, or a radical chosen from a hydroxyl radical and a linear or branched, saturated or unsaturated alkyl, perfluoroalkyl and/or hydrofluoroalkyl radical containing from 1 to 20 carbon atoms;
- R'₁, R"₁, R"₂ and R'''₁, which may be identical or different, represent a hydrogen atom, or a radical chosen from a linear or branched, saturated or unsaturated alkyl, perfluoroalkyl and/or hydrofluoroalkyl radical containing from 1 to 20 carbon atoms;
with the exclusion of compounds such that:
- R₁ and R₂ cannot simultaneously be a hydroxyl radical;
- R'''₁ and R'₂ cannot simultaneously be a hydroxyl radical;
- when S is a D-xylose and X is =CO, then R cannot be an unsubstituted phenyl;
- when S is a D-glucose and X is the function -OH, then R cannot be a p-hydroxyphenyl; and
- when S is a D-glucose and X is =O, then R cannot be a p-methoxyphenyl or a hexyl;
and at least one active agent for the hair chosen from anti-seborrhoeic agents; agents for combating squamous states of the scalp; active agents for stimulating hair regrowth and/or promoting the slowing down of hair loss, chosen from nicotinic acid esters, pyrimidine derivatives, agents that are both lipoxygenase inhibitors and clyclooxygenase inducers, and cyclooxygenase-inducing agents that promote hair regrowth; antibiotics; cinnarizine; nimodipine; nifedipine; estriol or the like; thyroxine and salts thereof; oxendolone, spironolactone, diethylstilbestrol and flutamide; cromakalim and nicorandil.
